# EUROPEAN PATENT SPECIFICATION

(11) **EP 3 475 698 B1**
(45) Date of publication and mention of the grant of the patent: **14.10.2020**
(21) Application number: 17748920.0
(22) Date of filing: 22.06.2017
(51) Int. Cl.: G01N 33/50

(54) **METHOD FOR RNA TAGGING AND ANALYSIS ON SINGLE CELL**
METHODE ZUM RNA TAGGING UND ZUR ANALYSE EINZELNER ZELLEN
METHODE POUR LE MARQUAGE ET L'ANALYSE DE L'ARN SUR DES CELLULES UNIQUES

(30) Priority: 23.06.2016 IT UA20164610
(43) Date of publication of application: 01.05.2019
(73) Proprietor: Centro Cardiologico Monzino SpA, 20121 Milano (MI) (IT)
(72) Inventor: CAMERA, Marina, 20121 Milano (IT); ZARA, Chiara, 20121 Milano (IT); TREMOLI, Elena, 20121 Milano (IT)
(74) Representative: Rigamonti, Dorotea
(86) International application number: PCT/IB2017/053733
(87) International publication number: WO 2017/221194

(56) References cited:
- US-A1- 2010 129 808
- WILLIAM E. BRILEY ET AL: "Quantification and real-time tracking of RNA in live cells using Sticky-flares", PROCEEDINGS NATIONAL ACADEMY OF SCIENCES PNAS, vol. 112, no. 31, 20 July 2015 (2015-07-20), pages 9591-9595, XP055360330, US ISSN: 0027-8424, DOI: 10.1073/pnas.1510581112
- M. BRAMBILLA ET AL: "Human megakaryocytes confer tissue factor to a subset of shed platelets to stimulate thrombin generation", THROMBOSIS AND HAEMOSTASIS, vol. 114, no. 3, 11 June 2015 (2015-06-11) , pages 579-592, XP055356367, DE ISSN: 0340-6245, DOI: 10.1160/TH14-10-0830 & M. Brambilla ET AL: "Supplementary Material to Brambilla et al. "Human megakaryocytes confer Tissue Factor to a subset", Thrombosis and Haemostasis, 11 June 2015 (2015-06-11), pages 1-19, XP055356373, Retrieved from the Internet: URL:https://th.schattauer.de/index.php?id= 4961 [retrieved on 2017-03-20]
- R. WOOLLEY ET AL: "From particle to platelet: Optimization of a stable, high brightness fluorescent nanoparticle based cell detection platform", NANOMEDICINE: NANOTECHNOLOGY, BIOLOGY AND MEDICINE, vol. 9, no. 4, 1 May 2013 (2013-05-01), pages 540-549, XP055356579, NL ISSN: 1549-9634, DOI: 10.1016/j.nano.2012.10.001
- MARIE-JOSÉE JACOBIN-VALAT ET AL: "Nanoparticles functionalised with an anti-platelet human antibody for in vivo detection of atherosclerotic plaque by magnetic resonance imaging", NANOMEDICINE: NANOTECHNOLOGY, BIOLOGY AND MEDICINE, vol. 11, no. 4, 1 May 2015 (2015-05-01), pages 927-937, XP055356576, NL ISSN: 1549-9634, DOI: 10.1016/j.nano.2014.12.006
- EBERBECK D ET AL: "Specific binding of magnetic nanoparticle probes to platelets in whole blood detected by magnetorelaxometry", JOURNAL OF MAGNETISM AND MAGNETIC MATERIALS, ELSEVIER, AMSTERDAM, NL, vol. 321, no. 10, 1 May 2009 (2009-05-01), pages 1617-1620, XP026052306, ISSN: 0304-8853, DOI: 10.1016/J.JMMM.2009.02.098 [retrieved on 2009-02-23]

## Description

### METHOD FOR RNA TAGGING AND ANALYSIS ON SINGLE CELL

The present invention relates to a method for RNA tagging and analysis on single cell, suitable for platelets, comprising the following steps:
a) Providing a population of cells of interest, wherein said cell population is a population of platelets;
b) Incubating said platelets for a time of 10 minutes to 3 hours, or 30 minutes to 2 hours, at a temperature from about 20 °C to about 37 °C, in a culture medium supplemented with a lipofection reagent and a SmartFlare™ probe of interest;
c) Fixing with a fixative;
d) Visualising and analysing the RNA of interest.

### Background art

Platelets are corpuscolar blood elements playing a crucial role in coagulation. The crucial role played by platelets in coagulation makes them key players in thrombotic phenomena. Recently, other functions have been attributed to platelets, such as, vascular integrity control, involvement in inflammatory and immune processes, tumour metastasis, angiogenesis and, last but not least, the onset and progression of atherothrombotic disease. The characteristic which distinguishes platelets is that they are core-free. Conversely, platelets possess granules, cytoplasmic organelles, and RNA.

Given the multifaceted role played by platelets and the presence of RNA therein, the need for tagging and analyzing the RNA contained therein is strongly felt. The SmartFlare™ RNA detection probe technology (Merck Millipore, Germany) allows for the analysis of intracellular RNA expression on single living cell. The SmartFlare™ method includes plating the cells, preferably in 96-well plates, at a 60-80% confluence. The SmartFlare™ probe is added to the culture and allowed to incubate for a period of about 16 hours, so as to allow it to enter the cells via endocytosis. Once inside the cell, the probe, recognizing a specific target, binds thereto and a subsequent fluorescence analysis reveals the presence thereof. Since platelets, as well as other cells with short half-life, such as monocytes, lymphocytes, granulocytes, may not be maintained in culture for a time as long as that required by the method, they are not suitable for the analysis by means of the above method.

The first attempts to introduce nucleic acids into platelets have been described by Hong W. et al. Transfection of Human Platelets with Short Interfering RNA, Clin Transl Sci. 2011; 4 (3): 180-182, though achieving a very low efficiency, equal to about 9%. Method improvements are described in WO 2014118817, with the purpose of using siRNA in platelets. However, no attempt is described about platelet RNA tagging, in particular, mRNA and miRNA, which tagging is thus not readily obtainable with the technologies available to date. In particular, a strong need for tagging RNA species modulated in acute pathology conditions is felt. William E. Briley et al. (PNAS 2015, 112:9591-9595) describe a method for RNA tagging on an immortalized cell line, HeLa cells.
US2010/129808 describes a method of determining the intracellular concentration of a target molecule comprising the step of contacting the target molecule with a nanoparticle.
Brambilla M. et al. (Thrombosis and Haemostasis 2015, 114:579-592) describe the megakaryocytes capability to transfer Tissue factor (TF) to platelets.
Wooley et al. (Nanomedicine: nanotechnology, biology and medicine 2013, 9:540-549) describe silica nanoparticles which are surface functionalised with PAMAM dendrimers to enable efficient conjugation to platelet activation-specific antibodies. receptor.
Jacobin-Valat Marie-Josée et al. (Nanomedicine: nanotechnology, biology and medicine 2015, 4:927-937) describe functionalized superparamagnetic iron oxide nanoparticles with a recombinant human antibody to target activated platelets.
Eberbeck D et al. (J. Magnetisms and magnetic materials 2009, 321: 1617-1620) describe the binding of monoclonal antibodies labelled with magnetic nanoparticles to CD61 surface proteins expressed by platelets.
Therefore, a method which allows to tag platelet RNA and cells with short half-life in vitro is needed, in order to overcome the limits imposed by the characteristics of the platelets themselves. Description of the invention

The present invention describes a method which surprisingly allows to overcome the limits imposed by the particular nature of platelets and cells with short half-life, thus leading to an efficient tagging of platelet RNA in vitro.

### Description of the drawings

Figure 1: platelet integrity analysis performed at the cytofluorimeter after treatment according to the Smartflare™ method (comparative).
Figure 2: platelet integrity analysis performed at the cytofluorimeter after treatment according to the method of the present invention.
Figure 3: comparative analysis of different lipofection reagents for the incorporation of the Smartflare™ probe, negative control (unflare) and positive control (uptake) in platelets.
Figure 4: Exemplary mRNA (18S and TF) expression levels obtained according to the method of the present invention.

### Detailed description of the invention:

It is herein described an innovative method for RNA tagging and analysis on single cell, suitable for platelets and cells with a short half-life, comprising the following steps:
a) Providing a population of cells of interest, wherein said cell population is a population of platelets;
b) Incubating said platelets for a time of 10 minutes to 3 hours, or 30 minutes to 2 hours, at a temperature from about 20 °C to about 37 °C, in a culture medium supplemented with a lipofection reagent and a SmartFlare™ probe of interest;
c) Fixing with a fixative;
d) Visualising and analysing the RNA of interest.

In a preferred embodiment, said RNA is mRNA or miRNA.

For the aim of the present invention, SmartFlare™ probes mean probes provided by Merck Millipore, which recognize mRNA or miRNA key target. Said probes are Cy5 or Cy3 tagged. However, by means of the methods known to those skilled in the art, further probes may be synthesised for specific purposes, i.e. probes recognizing targets of particular interest. For the aim of the present invention, SmartFlare™ probes are probes capable of entering a cell, recognizing a target RNA of interest, and making it detectable by the SmartFlare™ method; by way of not limiting example, the SmartFlare™ probes found in the Merck Millipore catalogue are included. Also included are those additional probes which may be synthesised by those skilled in the art for further targets and purposes of interest, in particular oligonucleotide sequences capable of pairing with target RNAs, such as by way of example DNA sequences, tagged with a fluorophore detectable by cytofluorimetry and microscopy. In a preferred embodiment, said method is operated on platelets. Preferably, said incubation takes place for about 1 hour at room temperature, or at about 37 °C. Preferably, said culture medium is RPMI1640 supplemented with glutamine or GlutaMAX™.

In a still more preferred embodiment, about 500,000 platelets resuspended in about 100 µ 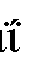 of culture medium are provided. Preferably, said lipofection reagent is selected from: TransIT- LT1 (Mirus), Turbofect transfection reagent (Invitrogen), RiboJuiceTM siRNA Transfection Reagent (Merck Millipore), NanoJuice® Transfection Kit (Merck Millipore), GeneJuice® Transfection Reagent (Merck Millipore) and other transfecting reagents known to those skilled in the art. The experimental data obtained and reported below showed that, despite the great issues, well known in the prior art, relating to the introduction of nucleic acids in platelets, it was surprisingly possible to achieve more than satisfactory transfection levels using TransIT-LT1 (Mirus), Gene Juice, Nano Juice, Ribo Juice (Merck Millipore) and Turbofect transfection reagent (Invitrogen) . From a morphological analysis, it has been observed that, upon transfection, the platelets do not exhibit major alterations.

1.5 µl of said lipofection reagents, 1:10 diluted, are added to 100 µl of the culture medium. Preferably, said culture medium contains 300 pM of said SmartFlare™ probe.

Preferably, said fixative used in said step c) is 1% paraformaldehyde (PFA).

Optionally, at the end of the incubation as described in step b) and before said fixing step c), said platelets are tagged with a specific antibody, for example FITC-tagged antiCD41. Thereby, platelets containing the RNA of interest may be detected.

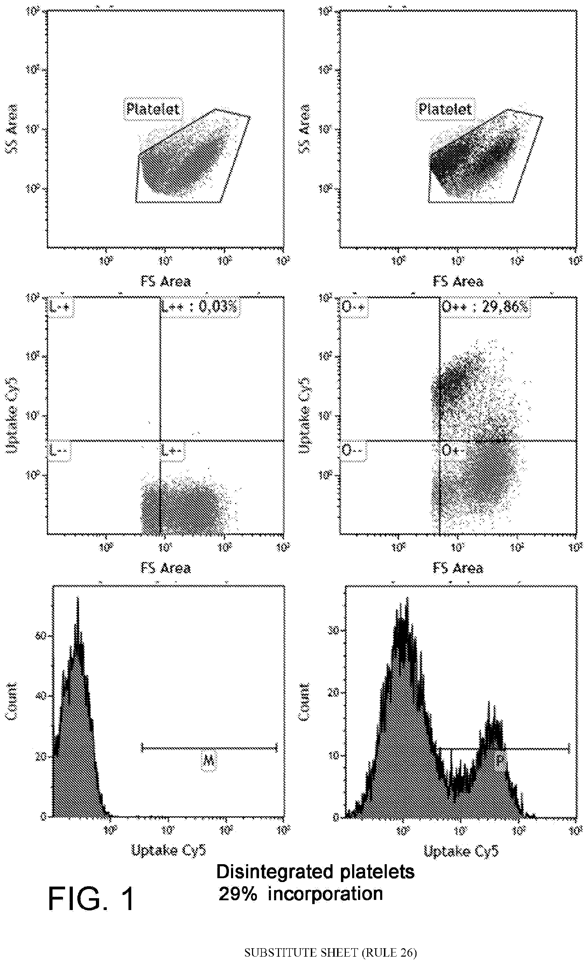

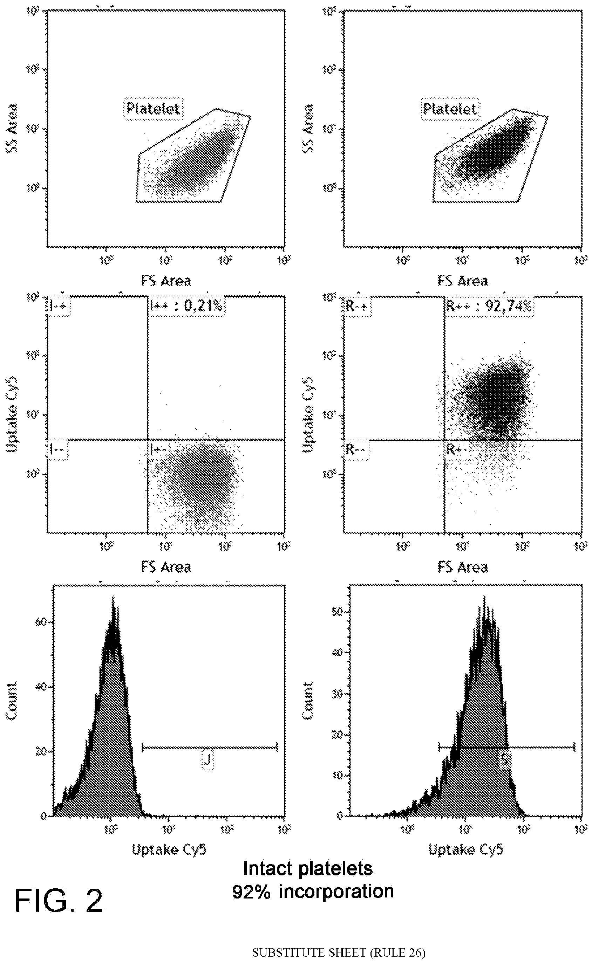

Figures 1 and 2 show platelet integrity data using the method as proposed by SmartFlare™ technology and the method according to the present invention, respectively. It is apparent that a marked morphological platelet alteration occurs by applying the method known from the prior art, thus making the method not applicable to the platelets, and that the method described herein surprisingly obviates such a morphological alteration and keeps the platelets intact and therefore sensitive to tagging. It is particularly efficient, for the purposes of the present invention, isolating platelets from whole blood by the method described below. The blood is collected by venous withdrawal from the cephalic vein of donors who have signed their informed consent to participate in the study. Whole blood (WB) was taken by means of a 19-gauge needle, without venous stasis, and placed in a tube containing citrate (1/10 of 0.129 M sodium citrate volume) and corn trypsin inhibitor (50 g/ml) (Vacutainer, Becton Dickinson) discarding the first 4 ml. For platelet preparation, WB was centrifuged at room temperature, in the absence of a brake, preferably at 100 g for 15'. For the aim of the present invention, the total platelet preparation analysed by the Sysmex XE-2100 Automated Hematology Analyzer is used to determine the platelet recovery, the platelet average volume (MPV), the platelet immature fraction (IPF), and the platelet distribution width (PDW) .

Some examples of results obtained by the method according to the present invention are shown hereinbelow. Said examples are not intended to limit in any manner the scope of the present invention to that specifically exemplified herein. In particular, the results obtained using probes for the Tissue Factor (TF mRNAs) and 18S in platelets are herein described. However, probes for further RNAs of specific interest may be synthesised and used according to methods known to those skilled in the art.

### EXAMPLES Example 1: Comparison of the incorporation efficiency of the SmartFlare™ probe

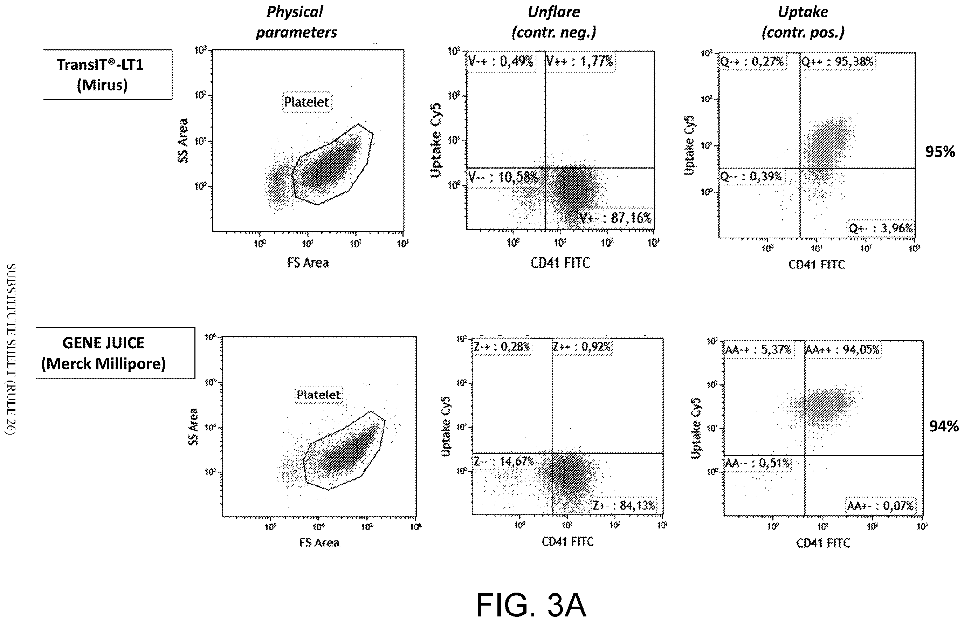

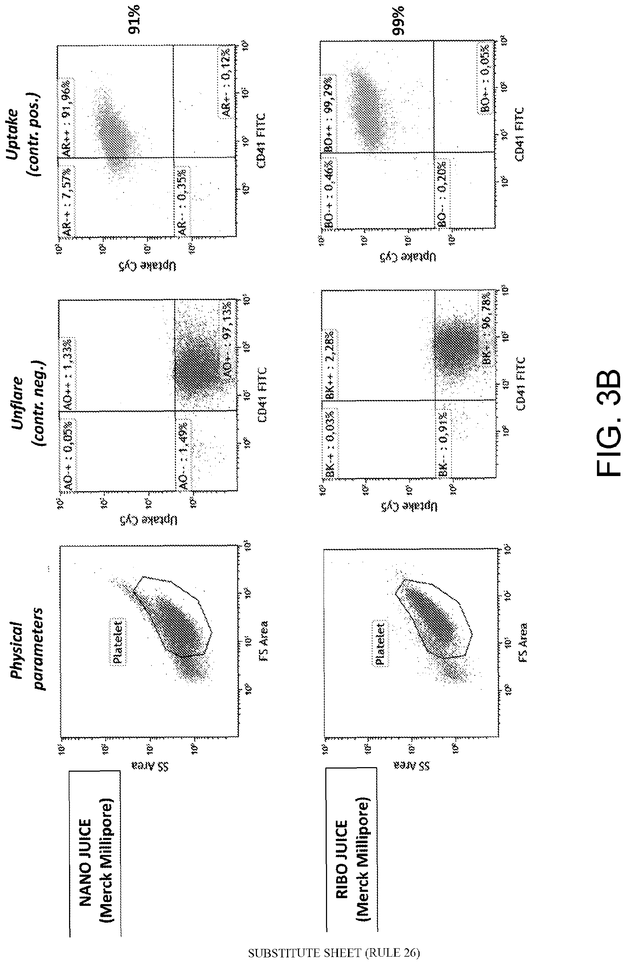

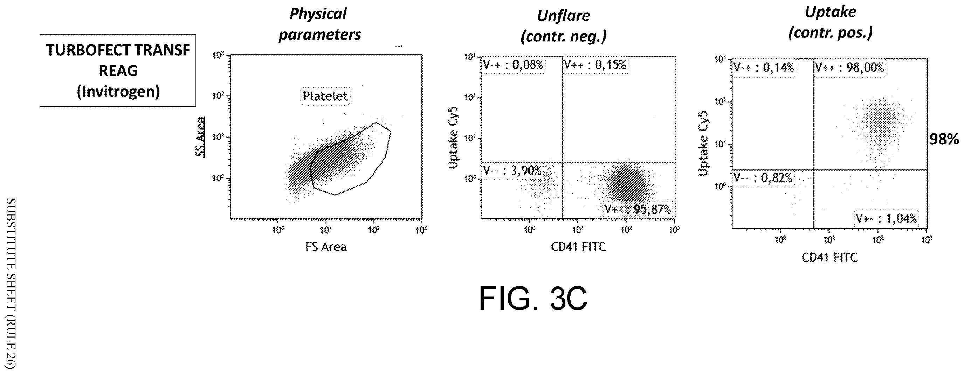

A platelet preparation was incubated with a SmartFlare™ probe for the uptake control. Each sample was incubated in the presence of one of the lipofection reagents shown in Figure 3 and 300 pM of the Uptake Cy5 probe and the negative control (Scramble Cy5) for 1 hour at room temperature in RPMI1640 culture medium supplemented with glutamine. By means of the Kaluza image analysis software, the fluorescence degree was quantified and percent values of the platelets expressing the positive control signal were obtained, by subtracting the signal from the negative control in order to remove the unspecified signal.

The results are shown in Figure 3. The reported data surprisingly show that the solution of the present invention can introduce nucleic acids in platelets, more specifically probes for detecting tagged RNA, thus surprisingly allowing a platelet RNA to be analysed on living single cell. It is worth noting the high efficiency obtained, as shown on the right in Figure 3, with each of the lipofection reagents reported.

### Example 2: Tagging and analysis of TF and 18S mRNA in platelets.

It is known that a subpopulation of human platelets express TF mRNA. The used probes include:
- Control probes: 18S-Hu-Cy5 Smartflare™ (Cat No. SF-142); negative control: Scramble-Cy5 SmartFlare™ (Cat No. SF-102) which binds non-sense mRNA sequences not present in the sample; positive control: Uptake-Cy5 SmartFlare™ (Cat No. SF-137) having a constitutively fluorescent fluorophore. - Specific probes were designed for mRNAs of interest, in particular a Cy5 tagged probe for TF mRNA, SEQ ID NO. 1 (GTTTCACACCTTACCTGGAGACAAACC) .

The platelets were isolated from whole blood of healthy volunteers after signature of the informed consent, according to methods known to those skilled in the art.

For each of the above probes, 500,000 platelets were incubated for 1 hour at room temperature in RPMI1640 culture medium supplemented with glutamine with 1.5 µl of transfection reagent Transit-LTl 1:10 diluted and 300 pM of one of the above SmartFlare™ probes.

After said incubation, the platelets were tagged with a FITC- tagged anti-CD41 antibody and fixed with 1% PFA. By cytofluorimetry, the Cy5 fluorescence of the probes was analysed in the CD41 positive platelets.

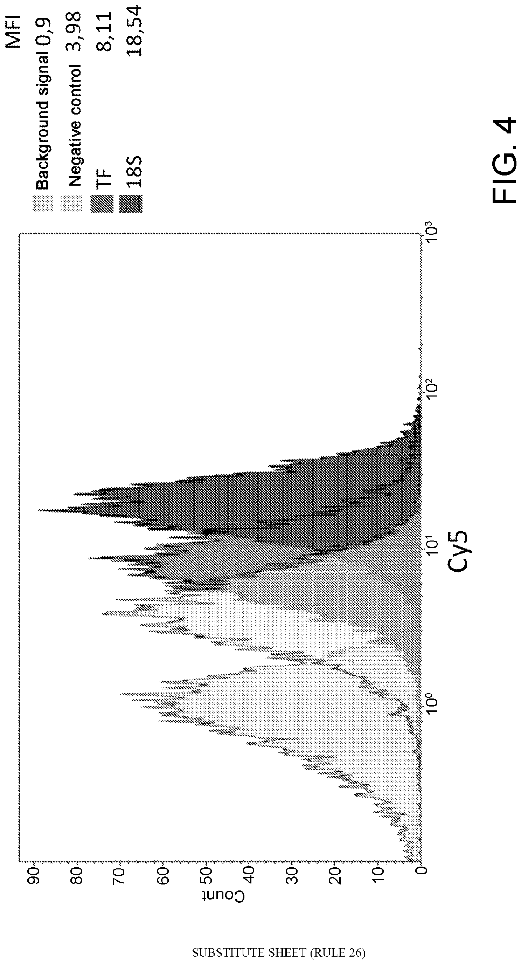

Once the platelet aggregates have been excluded, the signal from the positive control (Uptake Cy5) and the negative control (Scramble Cy5) was assessed. By means of the Kaluza analysis software, the median fluorescence intensity (MFI) of the probes which respectively detect 18S and TF mRNAs between the CD41 and Cy5 positive events was quantified, using Scramble Cy5 as the negative control to subtract the unspecified signal. The data are expressed as MFI and the results are shown in Figure 4. In particular, TF was found to be expressed at low levels in the platelets versus 18S which is very abundant.

## Claims

1. A method for RNA tagging and analysis on single cell comprising the following steps:
a) providing a population of cells of interest, wherein said cell population is a population of platelets;
b) incubating said platelets for a time of 10 minutes to 3 hours, or 30 minutes to 2 hours, at a temperature comprised in the range 20 °C - 37 °C, in a culture medium supplemented with a lipofection reagent and a SmartFlareTM probe of interest;
c) fixing with a fixative;
d) visualising and analysing the RNA of interest.

2. The method according to claim 1, wherein said RNA is mRNA and miRNA.

3. The method according to one of claims 1 or 2, wherein said incubation occurs for about 1 hour at room temperature in RPMI1640 culture medium.

4. The method according to one of claims 1 to 3, wherein approximately 500,000 platelets resuspended in about 100 µl of culture medium are provided.

5. The method according to claim 4, wherein 1.5 µl of lipofection reagent diluted 1:10 is added to 100 µl of said culture medium containing 300 pM of said SmartFlareTM probe.

6. The method according to one of claims 1 to 5, wherein said platelets are tagged with a platelet-specific antibody at the end of the incubation of step b) and before said fixing step c) .

## Patentansprüche

1. Verfahren zur Markierung und Analyse von RNA an einzelnen Zellen, umfassend die folgenden Schritte:
a) Bereitstellen einer zu testenden Zellkultur, wobei die Zellkultur eine Kultur von Blutplättchen ist;
b) Inkubieren der Blutplättchen für eine Dauer von 10 Minuten bis 3 Stunden oder 30 Minuten bis 2 Stunden bei einer Temperatur im Bereich von 20 °C bis 37 °C in einem Kulturmedium, welches versetzt ist mit einem Lipofektions-Reagens und einer SmartFlareTM Sonde von Interesse;
c) Fixieren mit einem Fixiermittel;
d) Visualisieren und Analysieren der RNA von Interesse.

2. Verfahren nach Anspruch 1, wobei es sich bei der RNA um mRNA und miRNA handelt.

3. Verfahren nach einem der Ansprüche 1 bis 2, wobei die Inkubation für etwa 1 Stunde bei Raumtemperatur in einem RPMI1640 Kulturmedium stattfindet.

4. Verfahren nach einem der Ansprüche 1 bis 3, wobei etwa 500,000 Blutplättchen, resuspendiert in etwa 100 µl des Kulturmediums, bereitgestellt werden.

5. Verfahren nach Anspruch 4, wobei 1.5 µL Lipofektions-Reagens in einer 1:10 Verdünnung zu 100 µl des Kulturmediums, welches 300 pM der SmartFlareTM Sonde enthält, zugegeben werden.

6. Verfahren nach einem der Ansprüche 1 bis 5, wobei die Blutplättchen mit einem Blutplättchen-spezifischen Antikörper am Ende des Inkubations-Schritts b) und vor dem Fixierungs-Schritt c) markiert werden.

## Revendications

1. Procédé d'étiquetage et d'analyse d'ARN sur cellule unique comprenant les étapes suivantes :
a) fourniture d'une population de cellules d'intérêt, ladite population de cellules étant une population de plaquettes ;
b) incubation desdites plaquettes pendant une durée de 10 minutes à 3 heures, ou 30 minutes à 2 heures, à une température comprise dans la plage de 20 °C à 37 °C, dans un milieu de culture supplémenté avec un réactif de lipofection et une sonde SmartFlare™ d'intérêt ;
c) fixation avec un fixateur ;
d) visualisation et analyse de l'ARN d'intérêt.

2. Procédé selon la revendication 1, dans lequel ledit ARN est un ARNm et un miARN.

3. Procédé selon l'une des revendications 1 ou 2, dans lequel ladite incubation est conduite pendant environ 1 heure à température ambiante dans du milieu de culture RPM11640.

4. Procédé selon l'une des revendications 1 à 3, dans lequel approximativement 500 000 plaquettes remises en suspension dans environ 100 µl de milieu de culture sont fournies.

5. Procédé selon la revendication 4, dans lequel 1,5 µl de réactif de lipofection dilué 1:10 est ajouté à 100 µl dudit milieu de culture contenant 300 pM de ladite sonde SmartFlare™.

6. Procédé selon l'une des revendications 1 à 5, dans lequel lesdites plaquettes sont étiquetées avec un anticorps spécifique aux plaquettes à la fin de l'incubation de l'étape b) et avant ladite étape de fixation c).
